# FASCICULE DE BREVET EUROPEEN

(11) **EP 0 803 242 B1**
(45) Date de publication et mention de la délivrance du brevet: **13.03.2002**
(21) Numéro de dépôt: 97400805.4
(22) Date de dépôt: 08.04.1997
(51) Int. Cl.: A61K 7/48, A61K 7/04, A61K 7/043

(54) **Utilisation de céramide pour le traitement des ongles**
Verwendung von Ceramiden zur Behandlung von Nägeln
Use of ceramides for the treatment of nails

(30) Priorité: 22.04.1996 FR 9605022
(43) Date de publication de la demande: 29.10.1997
(73) Titulaire: L'OREAL, 75008 Paris (FR)
(72) Inventeur: Ramin, Roland, 91760 Itteville (FR)
(74) Mandataire: Dodin, Catherine

(56) Documents cités:
- EP-A- 0 587 288
- EP-A- 0 608 600
- EP-A- 0 647 617
- EP-A- 0 679 383
- WO-A-92/06982

## Description

La présente invention a pour objet l'utilisation, dans une composition cosmétique, d'au moins un composé de type céramide pour le traitement cosmétique de l'ongle.

Il est bien connu que les ongles présentent, de façon fréquente, des défauts de structure et de consistance, ceux-ci pouvant être d'origine diverses et notamment liés au fonctionnement interne de l'individu, à ses conditions de vie, à son mode d'alimentation, à son âge, à ses états de fatigue ou de surmenage.
Ces défauts peuvent également apparaître sous l'effet d'actions qui érodent, à la suite par exemple d'expositions prolongées ou répétées à des agents détergents, à des solvants, à des produits chimiques en particulier ménager, à des atmosphères chaudes ou froides, humides ou sèches, ou à des expositions aux rayonnements UV.
Ces défauts de structure et de consistance ont pour effet de rendre la surface des ongles inesthétique, ce qui peut être source de gêne et de désagréments multiples.

En vue de renforcer les ongles, on a déjà proposé différents types de composition essentiellement basée sur l'emploi soit d'agents réticulants des protéines destinées à renforcer le réseau kératinique comme par exemple le formol, soit d'agents à fonction essentiellement nutritive tels que par exemple la cystine, le cholestérol, la S-carboxyméthylcystéïne ou encore des extraits de collagène.
L'utilisation de tels agents réticulants ou de tels agents à fonction nutritive ne permet pas cependant d'obtenir de bons résultats et présente par ailleurs certains inconvénients. En particulier, les produits à base de formol peuvent provoquer certaines réactions d'allergie.

Les documents WO-A-92/06982 et EP 0 647 617 décrivent des compositions cosmétiques comprenant des pseudocéramides ou des céramides applicables sur les ongles et permettant d'obtenir un effet hydratant.

Le demandeur a constaté, de façon surprenante et inattendue, qu'en utilisant des composés de type céramides, il était possible d'améliorer l'état de surface des ongles. En particulier, la surface externe de l'ongle traitée par l'application d'une composition comprenant un composé de type céramide permet d'obtenir une surface externe de l'ongle plus homogène : l'ongle devient plus lisse et plus brillant.

La présente invention a donc pour objet l'utilisation, dans une composition cosmétique, d'au moins un composé de type céramide pour uniformiser la surface externe des ongles.
En particulier, le céramide peut améliorer le caractère lisse et/ou brillant de la surface externe des ongles.
L'application de céramides sur l'ongle conformément à l'invention est particulièrement avantageuse pour le traitement des ongles striés. On remarque que le traitement de tels ongles permet d'obtenir une bonne amélioration de la surface externe de l'ongle puisque cette surface traitée est bien plus uniforme.

Par "uniformiser la surface externe de l'ongle", on entend que le composé de type céramide forme un dépôt uniforme par adsorption sur la surface externe de l'ongle. Sans être tenu par la présente explication, on peut envisager que ceci est dû au fait que le céramide diffuse et pénètre dans les espaces intercellulaires de l'ongle, d'où un ongle d'aspect plus lisse et plus brillant.

Selon la présente invention, on entend, par composé de type céramide, les céramides et/ou les glycocéramides et/ou les pseudocéramides. Ils sont choisis de préférence parmi les molécules naturelles ou synthétiques répondant à la formule (I) suivante 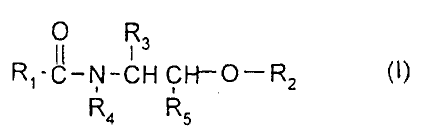 dans laquelle :
- R₁ désigne :
   - soit un radical hydrocarboné, linéaire ou ramifié, saturé ou insaturé, en C₁-C₅₀, de préférence en C₅-C₅₀, et plus préférentiellement en C₉-C₃₀, ce radical pouvant être substitué par un ou plusieurs groupements hydroxyle éventuellement estérifiés par un acide R₇COOH, R₇ étant un radical hydrocarboné, saturé ou insaturé, linéaire ou ramifié, éventuellement mono ou polyhydroxylé, en C₁-C₃₅ et de préférence en C₁₆-C₃₀, le ou les hydroxyles du radical R₇ pouvant être estérifiés par un acide gras saturé ou insaturé, linéaire ou ramifié, éventuellement mono ou polyhydroxylé, en C₁-C₃₅,
   - soit un radical R"-(NR-CO)-R', R désigne un atome d'hydrogène ou un radical hydrocarboné C₁-C₂₀, de préférence C₁-C₁₀, mono ou polyhydroxylé, préférentiellement monohydroxylé, R' et R" sont des radicaux hydrocarbonés dont la somme des atomes de carbone est comprise entre 9 et 30, R' étant un radical divalent,
   - soit un radical R₈-O-CO-(CH₂)ₚ, R₈ désignant un radical hydrocarboné en C₁-C₂₀, p étant un entier variant de 1 à 12 ;
- R₂ est choisi parmi un atome d'hydrogène, un radical de type saccharidique, en particulier un radical (glycosyle)ₙ, (galactosyle)ₘ ou sulfogalactosyle, un résidu de sulfate ou de phosphate, un radical phosphoryléthylamine et un radical phosphoryléthylammonium, dans lesquels n est un entier variant de 1 à 4 et m est un entier variant de 1 à 8 ; +
- R₃ désigne un atome d'hydrogène ou un radical hydrocarboné en C₁-C₃₃, de préférence C₁₆-C₂₇, saturé ou insature, hydroxylé ou non, le ou les hydroxyles pouvant être estérifiés par un acide minéral ou un acide R₇COOH, R₇ ayant les mêmes significations que ci-dessus, le ou les hydroxyles pouvant être éthérifiés par un radical (glycosyle)ₙ, (galactosyle)ₘ, sulfogalactosyle, phosphoryléthylamine ou phosphoryléthylammonium, R, pouvant également être substitué par un ou plusieurs radicaux alkyle en C₁-C₁₄ ;
   de préférence, R₃ désigne un radical α-hydroxyalkyle en C₁₅-C₂₆, le groupement hydroxyle étant éventuellement estérifié par un α-hydroxyacide en C₁₆-C₃₀ ;
- R₄ désigne un atome d'hydrogène, un radical méthyle, éthyle, un radical hydrocarboné en C₃-C₅₀, de préférence en C₁₆-C₂₇, saturé ou insaturé, linéaire ou ramifié, éventuellement hydroxylé ou un radical -CH₂-CHOH-CH₂-O-R₆ dans lequel R₆ désigne un radical hydrocarboné en C₁₀-C₂₆ ou un radical R₈-O-CO-(CH₂)ₚ, R₈ désigne un radical hydrocarboné en C₁-C₂₀, p étant un entier variant de 1 à 12,
- R₅ désigne un atome d'hydrogène ou un radical hydrocarboné en C₁-C₃₀ saturé ou insaturé, linéaire ou ramifié, éventuellement mono ou polyhydroxylé, le où les hydroxyles pouvant être éthérifiés par un radical (glycosyle)ₙ, (galactosyle)ₘ, sulfogalactosyle, phosphoryléthylamine ou phosphoryléthylammonium ; de préférence, R₅ désigne un radical hydrocarboné en C₁-C₄ saturé ou insaturé, linéaire ou ramifié ;
sous réserve que lorsque R₃ et R₅ désignent hydrogène ou lorsque R₃ désigne hydrogène et R₅ désigne méthyle alors R₄ ne désigne pas un atome d'hydrogène, un radical méthyle ou éthyle.

Parmi les composés de formule (I) ci-dessus, on préfère les céramides et/ou glycocéramides décrits par DOWNING dans Journal of Lipid Research, Vol. 35, page 2060, 1994 ou ceux décrits dans la demande de brevet français FR-2 673 179, et dont les enseignements sont ici inclus à titre de référence.

Les composés de type céramide plus particulièrement préférés selon l'invention sont les composés de formule (I) pour lesquels R₁ désigne un radical alkyle saturé ou insaturé dérivé d'acides gras en C₁₄-C₂₂, de préférence en C₁₆-C₂₂, éventuellement hydroxylé; R₂ désigne un atome d'hydrogène ; et R₃ désigne un radical linéaire saturé en C₁₁-C₁₇ éventuellement hydroxylé et de préférence en C₁₃-C₁₅ et plus préférentiellement en C₁₅.

De tels composés sont par exemple :
- le 2-N-linoléoylamino-octadécane-1,3-diol,
- le 2-N-oléoylamino-octadécane-1,3-diol,
- le 2-N-palmitoylamino-octadécane-1,3-diol,
- le 2-N-stéaroylamino-octadécane-1,3-diol,
- le 2-N-béhénoylamino-octadécane-1,3-diol,
- le 2-N-[2-hydroxy-palmitoyl]-amino-octadécane-1,3-diol,
- le 2-N-stéaroyl amino-octadécane-1,3,4 triol et en particulier la N-stéaroyl phytosphingosine,
- le 2-N-palmitoylamino-hexadécane-1,3-diol
ou les mélanges de ces composés.

On peut aussi utiliser des mélanges spécifiques tels que par exemple les mélanges de céramide(s) 2 et de céramide(s) 5 selon la classification de DOWNING.

On peut également utiliser les composés de formule (I) pour lesquels R₁ désigne un radical alkyle saturé ou insaturé dérivé d'acides gras ; R₂ désigne un radical galactosyle ou sulfogalactosyle ; et R₃ désigne un radical hydrocarboné en C₁₂-C₂₂, saturé ou insaturé et de préférence un groupement -CH-CH-(CH₂)₁₂-CH₃₋

Des composés de type céramide sont par exemple décrits dans les demandes de brevet DE4424530, DE4424533, DE4402929, DE4420736, WO95/23807, WO94/07844, EP-A-0646572, WO95/16665, FR-2 673 179, EP-A-0227994 et WO 94/07844, WO94/24097, WO94/10131 dont les enseignements sont ici inclus à titre de référence.

A titre d'exemple, on peut citer le produit constitué d'un mélange de glycocéramides, vendu sous la dénomination commerciale GLYCOCER par la société WAITAKI INTERNATIONAL BIOSCIENCES.

On peut également utiliser les composés décrits dans les demandes de brevet EP-A-0 227 994, EP-A-0 647 617, EP-A-0 736 522 et WO 94 / 07 844.

De tels composés sont par exemple le QUESTAMIDE H, encore appelé bis-(N-hydroxyéthyl N-cétyl) malonamide et vendu par la société QUEST et le N-(2-hydroxyéthyl)-N-(3-cétyloxy-2-hydroxypropyl)amide d'acide cétylique.

On peut également utiliser le N-docosanoyl N-méthyl-D-glucamine tel que décrit dans la demande de brevet WO 94 / 24097 et le N-dodécasanoyl N-méthyl-D-glucamine tel que décrit dans la demande de brevet WO 92 / 05 764.

De préférence, le composé de type céramide utilisé dans la présente invention est choisi parmi la N-linoléoyldihydrosphingosine, la N-oléoyldihydrosphingosine, la N-palmitoyldihydrosphingosine, la N-stéaroyldihydrosphingosine, la N-béhénoyldihydrosphingosine, la N-2-hydroxypalmitoyldihydrosphingosine, la N-stéaroylphytosphingosine, ou les mélanges de ces composés, et avantageusement parmi la N-oléoyldihydrosphingosine, la N-2-hydroxypalmitoyldihydrosphingosine et la N-stéaroylphytosphingosine.

Selon l'invention, la teneur en composé de type céramide peut aller de 0,01 % à 10 % en poids, et de préférence de 0,1 % à 1 %, par rapport au poids total de la composition.

Le composé de type céramide peut être incorporé dans la composition sous forme de poudre ou sous forme dispersé dans l'eau, un solvant organique tel que l'isopropanol, ou une huile.

Par ailleurs, la composition cosmétique de l'invention comprend un support cosmétiquement acceptable.
Ce support peut comprendre des solvants organiques, de l'eau, et/ou est un milieu huileux, par exemple.

Comme solvants organiques, on peut citer les cétones, comme l'acétone, la méthyl-éthyl-cétone, la méthyl-isobutyl-cétone; les éthers de glycols; les alcools comme l'éthanol, le n-butanol, le n-propanol, l'isopropanol; les acétates comme l'acétate de butyle, d'éthyle, d'isopropyle, l'acétate de méthoxy-2-éthyle; les hydrocarbures linéaires ou ramifiés tels que l'hexane ou l'octane; ou encore les hydrocarbures aromatiques tels que le xylène et le toluène,

Lorsque le support cosmétiquement acceptable comprend de l'eau, la composition peut se présenter notamment sous la forme d'une solution aqueuse ou hydroalcoolique, d'une émulsion huile-dans-eau ou eau-dans-huile, voire d'une émulsion multiple, ou encore sous la forme d'un gel aqueux.

Ledit milieu huileux peut comprendre une ou plusieurs huiles, volatiles et/ou non volatiles, par exemple d'origine végétale, minérale, animale et/ou de synthèse, parmi lesquelles on peut citer:
. les huiles animales ou végétales formées par des esters d'acide gras et de polyols, en particulier les triglycérides liquides, par exemple les huiles de tournesol, de maïs, de soja, de courge, de pépins de raisin, de sésame, de noisette, d'abricot, d'amandes ou d'avocat, les huiles de poisson, le tricaprocaprylate de glycérol, ou les huiles végétales ou animales de formule R₁COOR₂ dans laquelle R₁ représente le reste d'un acide gras supérieur comportant de 7 à 19 atomes de carbone et R₂ représente une chaîne hydrocarbonée ramifiée contenant de 3 à 20 atomes de carbone, par exemple, l'huile de Purceilin ;
. les huiles essentielles naturelles ou synthétiques telles que, par exemple, les huiles d'eucalyptus, de lavandin, de lavande, de vétiver, de citron, de santal, de romarin, de camomille, de sarriette, de noix de muscade, de cannelle, d'hysope, de carvi, d'orange, de géraniol, de cade et de bergamote;
. les hydrocarbures, tels que l'hexadécane et l'huile de paraffine ;
. les esters d'acide minéral et d'un alcool ;
. les éthers et les polyéthers ;
. les huiles et gommes de silicones.

De plus, la composition peut comprendre un polymère filmogène, ce qui permet de déposer, par exemple sur l'ongle, un film résistant qui assure un contact prolongé du céramide avec la surface de l'ongle.

A titre d'exemple, le polymère peut être choisi parmi la nitrocellulose, l'acétobutyrate de cellulose, les butyralpolyvinyliques, les résines alkydes, les polyesters, les polyuréthannes, les polyester-polyuréthanes, les polyéther-polyuréthanes, les polymères radicalaires, notamment de type acrylique, acrylique styrène et/ou vinylique et leurs mélanges.
Les polymères peuvent être dissous ou dispersés dans la composition. Ils peuvent être généralement présents à une teneur allant de 1 % à 40 % en poids par rapport au poids total de la composition.

La composition selon l'invention peut également comprendre, en plus du polymère filmogène, des plastifiants qui permettent de régler la flexibilité du film sans affaiblir sa résistance physique.
Les plastifiants utilisables sont ceux couramment employés dans les compositions de vernis à ongles. Comme plastifiants, on peut citer les phtalates de dibutyle, de dioctyle, de di-isobutyle, de diméthoxyéthyl, les benzoates de benzyle, de glycéryle ; les citrates de triéthyle, de tributyle, l'acétyl-citrate de tributyle ; les phosphates de tributyle, de triphényle; les glycols ; le camphre ainsi que leurs dérivés et leurs mélanges. Les plastifiants peuvent être généralement présents à une teneur allant de 1 % a 30 % en poids par rapport au poids total de la composition.

La composition selon l'invention peut , en outre, comprendre des agents rhéologiques. Comme agents rhéologiques, on peut citer les argiles, la silice pyrogénée, les polymères associatifs de type polyuréthane, les dérivés cellulosiques, les gommes naturelles telles que la gomme de xanthane.

Par ailleurs, la composition selon l'invention peut contenir des adjuvants couramment utilisés dans les compositions cosmétiques. On peut citer à titre d'exemple d'adjuvants les colorants, les pigments, les nacres, les laques, les agents anti-UV, les agents épaississants, les tensioactifs, les cires, les parfums, et des actifs de tels que le D-panthénol, le phytantriol, les vitamines et leurs dérivés, la kératine et ses dérivés, la mélanine, le collagène, la cystine, le chitosane et ses dérivés, la biotine, les oligo-éléments, la glycérine, les hydrolysats de protéines, les phospholipides, les agents hydratants. Bien entendu, l'homme du métier veillera à choisir ce ou ces éventuels adjuvants, et/ou leur quantité, de telle manière que les propriétés avantageuses de la composition selon l'invention ne soient pas, ou substantiellement pas, altérées par l'adjonction envisagée.

La composition selon l'invention peut se présenter sous forme d'un vernis à ongles ou d'une composition de soin des ongles

On va maintenant donner des exemples illustrant la présente invention.

### Exemple 1 :

On a préparé une base brillante de soin des ongles ayant la composition suivante:
- Nitrocellulose 10 g
- Résine et plastifiant 15 g
- N-oléoyldihydrosphingosine 0, 5 g
- Colorants et additifs 0, 5 g
- solvants (acétates d'éthyle et de butyle, alcool isopropylique) qsp 100 g

On a ainsi obtenu une base brillante de soin qui s'applique aisément sur l'ongle. Cette base permet, après séchage, l'obtention d'un film lisse, homogène et brillant.

### Exemple 2 :

On a préparé une base de soin des ongles ayant la composition suivante :
- Polymère acrylique en dispersion aqueuse 14 g
- Polyuréthanne en dispersion aqueuse 13, 5 g
- N-oléoyldihydrosphingosine 0, 5 g
- Cosolvants (alcool éthylique, alcool isopropylique) 6 g
- Colorants et additifs 0, 5 g
- Eau qsp 100 g

L'application de cette base de soin sur l'ongle laisse, après séchage, un film lisse, brillant et homogène.

### Exemple 3 :

On a préparé une base de soin des ongles ayant la composition suivante :
- Huile végétale 2 g
- N-oléoyldihydrosphingosine 0, 5 g
- Huile de silicone volatile 87, 5 g
- Solvants (alcool éthylique, alcool isobutylique) 10 g

On a ainsi obtenu une huile de soin pour les ongles qui, après application, dépose un film protecteur de l'ongle. Le film pénètre par massage au niveau de la matrice et de la tablette unguéale.

### Exemple 4 :

On a préparé une base brillante de soin des ongles ayant la composition suivante:
- Nitrocellulose 10 g
- Résine et plastifiant 15 g
- N-2-hydroxypalmitoyldihydrosphingosine 0, 5 g
- Colorants et additifs 0, 5 g
- solvants (acétates d'éthyle et de butyle, alcool isopropylique) qsp 100 g

On a ainsi obtenu une base brillante de soin qui s'applique aisément sur l'ongle. Cette base permet, après séchage, l'obtention d'un film lisse, homogène et brillant.

### Exemple 5 :

On a préparé une base de soin des ongles ayant la composition suivante :
- Huile végétale 2 g
- N-oléoyldihydrosphingosine 0, 47 g
- N-2-hydroxypalmitoyldihydrosphingosine 0, 02 g
- N-stéaroylphytosphingosine 0, 01 g
- Huile de silicone volatile 87, 5 g
- Solvants (alcool isopropylique) 10 g

On a ainsi obtenu une huile de soin pour les ongles qui, après application, dépose un film protecteur de l'ongle. Le film pénètre par massage au niveau de la matrice et de la tablette unguéale. L'ongle traité avec l'huile de soin devient lisse et brillant. La surface de l'ongle ainsi traité devient uniforme.

### Exemple 6 :

On a préparé une base de soin des ongles ayant la composition suivante :
- Huile végétale 2 g
- N-oléoyldihydrosphingosine 0, 4 g
- N-2-hydroxypalmitoyldihydrosphingosine 0, 1 g
- Alcool isopropylique 10 g
- Huile de silicone volatile qsp 100 g

On a ainsi obtenu une huile de soin pour les ongles qui, après application, dépose un film protecteur de l'ongle. Le film pénètre par massage au niveau de la matrice et de la tablette unguéale. L'ongle traité avec l'huile de soin devient lisse et brillant. La surface de l'ongle ainsi traité devient uniforme.

## Revendications

1. Utilisation, dans une composition cosmétique, d'au moins un composé de type céramide pour uniformiser la surface externe des ongles.

2. Utilisation selon la revendication 1, pour améliorer le caractère lisse de la surface externe des ongles.

3. Utilisation selon la revendication 1 ou 2, pour améliorer le caractère brillant de la surface externe des ongles.

4. Utilisation selon l'une des revendications 1 à 3, **caractérisée par le fait que** le composé de type céramide est un composé de formule (I) suivante : 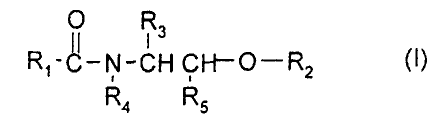 dans laquelle :
- R₁ désigne :
- soit un radical hydrocarboné, linéaire ou ramifié, saturé ou insaturé, en C₁-C₅₀, de préférence en C₅-C₅₀, ce radical pouvant être substitué par un ou plusieurs groupements hydroxyle éventuellement estérifiés par un acide R₇COOH, R₇ étant un radical hydrocarboné, saturé ou insaturé, linéaire ou ramifié, éventuellement mono ou polyhydroxylé, en C₁-C₃₅ , le ou les hydroxyles du radical R₇ pouvant être estérifiés par un acide gras saturé ou insaturé, linéaire ou ramifié, éventuellement mono ou polyhydroxylé, en C₁-C₃₅,
- soit un radical R"-(NR-CO)-R', R désigne un atome d'hydrogène ou un radical hydrocarboné C₁-C₂₀ mono ou polyhydroxylé, préférentiellement monohydroxylé, R' et R" sont des radicaux hydrocarbonés dont la somme des atomes de carbone est comprise entre 9 et 30, R' étant un radical divalent,
- soit un radical R₈-O-CO-(CH₂)ₚ, R₈ désignant un radical hydrocarboné en C₁-C₂₀, p étant un entier variant de 1 à 12 ;
- R₂ est choisi parmi un atome d'hydrogène, un radical de type saccharidique, en particulier un radical (glycosyle)ₙ, (galactosyle)ₘ ou sulfogalactosyle, un résidu de sulfate ou de phosphate, un radical phosphoryléthylamine et un radical phosphoryléthylammonium, dans lesquels n est un entier variant de 1 à 4 et m est un entier variant de 1 à 8 ;
- R₃ désigne un atome d'hydrogène ou un radical hydrocarboné en C₁-C₃₃, saturé ou insaturé, hydroxylé ou non, le ou les hydroxyles pouvant être estérifiés par un acide minéral ou un acide R₇COOH, R₇ ayant les mêmes significations que ci-dessus, le ou les hydroxyles pouvant être éthérifiés par un radical (glycosyle)ₙ, (galactosyle)ₘ, sulfogalactosyle, phosphoryléthylamine ou phosphoryl-éthylammonium, R₃ pouvant également être substitué par un ou plusieurs radicaux alkyle en C₁-C₁₄ ;
- R₄ désigne un atome d'hydrogène, un radical méthyle, éthyle, un radical hydrocarboné en C₃-C₅₀, saturé ou insaturé, linéaire ou ramifié, éventuellement hydroxylé ou un radical -CH₂-CHOH-CH₂-O-R₆ dans lequel R₆ désigne un radical hydrocarboné en C₁₀-C₂₆ ou un radical R₈-O-CO-(CH₂)ₚ, R₈ désigne un radical hydrocarboné en C₁-C₂₀, p étant un entier variant de 1 à 12,
- R₅ désigne un atome d'hydrogène ou un radical hydrocarboné en C₁-C₃₀ saturé ou insaturé, linéaire ou ramifié, éventuellement mono ou polyhydroxylé, le ou les hydroxyles pouvant être éthérifiés par un radical (glycosyle)ₙ, (galactosyle)ₘ, sulfogalactosyle, phosphoryléthylamine ou phosphoryléthylammonium,
sous réserve que lorsque R₃ et R₅ désignent hydrogène ou lorsque R₃ désigne hydrogène et R₅ désigne méthyle alors R₄ ne désigne pas un atome d'hydrogène, un radical méthyle ou éthyle.

5. Utilisation selon la revendication 4, **caractérisée par le fait que** le composé de type céramide est un composé de formule (I) dans laquelle
- R₁ désigne soit un radical hydrocarboné, linéaire ou ramifié, saturé ou insaturé en C₉-C₃₀, ce radical pouvant être substitué par un ou plusieurs groupements hydroxyle, ces groupements hydroxyle étant éventuellement estérifiés par un acide gras saturé ou insaturé en C₁₆-C₃₀ ; soit un radical R"-(NR-CO)-R', où R désigne un atome d'hydrogène ou un radical hydrocarboné en C₁-C₁₀ mono ou polyhydroxylé, préférentiellement monohydroxylé, R' et R" sont des radicaux hydrocarbonés dont la somme des atomes de carbone est comprise entre 9 et 30, R' étant un radical divalent ;
- R₂ désigne un atome d'hydrogène ou un radical (glycosyle)ₙ, (galactosyle)ₘ ou sulfogalactosyle, dans lesquels n est un nombre entier variant de 1 à 4 inclusivement et m est un nombre entier variant de 1 à 8 inclusivement ;
- R₃ désigne un atome d'hydrogène ou un radical hydrocarboné en C₁₆-C₂₇, saturé ou insaturé, ce radical pouvant être substitué par un ou plusieurs radicaux alkyle en C₁-C₁₄ ; R₃ peut également désigner un radical α-hydroxyalkyle en C₁₅-C₂₆ dont le groupement hydroxyle peut éventuellement être estérifié par un α-hydroxyacide en C₁₆-C₃₀ ;
- R₄ désigne un atome d'hydrogène, un radical hydrocarboné en C₁₆-C₂₇, saturé ou insaturé, ou un radical -CH₂-CHOH-CH₂-O-R₆ dans lequel R₆ désigne un radical hydrocarboné en C₁₀-C₂₆ ;
- R₅ désigne un atome d'hydrogène ou un radical hydrocarboné en C₁-C₄ mono ou polyhydroxylé.

6. Utilisation selon la revendication 4 , **caractérisée par le fait que** le composé de type céramide est un composé de formule (I) dans laquelle R₁ désigne un radical alkyle saturé ou insaturé dérivé d'acides gras en C₁₆-C₂₂ éventuellement hydroxylé ; R₂ désigne un atome d'hydrogène ; et R₃ désigne un radical linéaire saturé en C₁₅ éventuellement hydroxylé

7. Utilisation selon la revendication 6, **caractérisée par le fait que** le composé de type céramide est choisi parmi :
- la N-linoléoyldihydrosphingosine,
- la N-oléoyldihydrosphingosine,
- la N-palmitoyldihydrosphingosine,
- la N-stéaroyldihydrosphingosine,
- la N-béhénoyldihydrosphingosine,
- la N-2-hydroxypalmitoyldihydrosphingosine,
- la N-stéaroylphytosphingosine,
ou les mélanges de ces composés.

8. Utilisation selon la revendication 7, **caractérisée par le fait que** le composé de type céramide est choisi parmi la N-oléoyldihydrosphingosine, la N-2-hydroxypalmitoyl-dihydrosphingosine et la N-stéaroylphytosphingosine.

9. Utilisation selon l'une quelconque des revendications précédentes, **caractérisée par le fait que** la composition comprend une teneur en composé de type céramide de 0,01 % à 10 % en poids.

10. Utilisation selon l'une quelconque des revendications précédentes, **caractérisée par le fait que** la composition comprend une teneur en composé de type céramide de 0,1 % à 1 % en poids

11. Utilisation selon l'une quelconque des revendications précédentes, **caractérisée par le fait que** la composition comprend un support cosmétiquement acceptable choisi parmi les solvants organiques, l'eau et/ou les huiles.

12. Utilisation selon l'une quelconque des revendications précédentes, **caractérisée par le fait que** la composition comprend un polymère filmogène choisi parmi la nitrocellulose, l'acétobutyrate de cellulose, les butyralpoly vinyliques, les résines alkydes, les polyesters, les acryliques, les polyuréthannes.

13. Utilisation selon la revendication 12, **caractérisée par le fait que** la composition comprend un agent plastifiant.

14. Utilisation selon l'une quelconque des revendications précédentes, **caractérisée par le fait que** la composition se présente sous forme de composition à appliquer sur l'ongle telle qu'un vernis à ongles ou une composition de soin des ongles.

## Patentansprüche

1. Verwendung mindestens einer Verbindung vom Ceramidtyp in einer kosmetischen Zusammensetzung, um die äußere Oberfläche der Nägel gleichförmig zu machen.

2. Verwendung nach Anspruch 1, um die Glätte der äußeren Oberfläche der Nägel zu verbessern.

3. Verwendung nach Anspruch 1 oder 2, um den Glanz der äußeren Oberfläche der Nägel zu verbessern.

4. Verwendung nach einem der Ansprüche 1 bis 3, **dadurch gekennzeichnet, daß** die Verbindung vom Ceramidtyp eine Verbindung der folgenden Formel (I) ist: 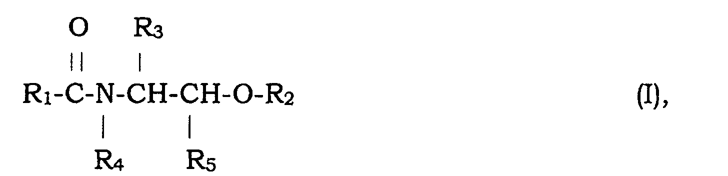 worin bedeuten:
- R₁
- entweder eine geradkettige oder verzweigte, gesättigte oder ungesättigte C₁₋₅₀-Kohlenwasserstoffgruppe und vorzugsweise C₅₋₅₀-Kohlenwasserstoffgruppe, wobei die Gruppe mit einer oder mehreren Hydroxygruppen substituiert sein kann, die gegebenenfalls mit einer Säure R₇COOH verestert sind, wobei R₇ eine gesättigte oder ungesättigte, geradkettige oder verzweigte C₁₋₃₅-Kohlenwasserstoffgruppe ist, die gegebenenfalls eine oder mehrere Hydroxygruppen aufweist, wobei die Hydroxygruppe(n) der Gruppe R₇ mit einer gesättigten oder ungesättigten, geradkettigen oder verzweigten, gegebenenfalls ein- oder mehrfach hydroxylierten C₁₋ ₃₅-Fettsäure verestert sein kann (können);
- oder eine Gruppe R"-(NR-CO)-R', wobei R Wasserstoff oder eine C₁₋₂₀-Kohlenwasserstoffgruppe mit einer oder mehreren Hydroxygruppen und vorzugsweise einer Hydroxygruppe bedeutet und R' und R" Kohlenwasserstoffgruppen sind, deren Summe an Kohlenstoffatomen im Bereich von 9 bis 30 liegt, wobei R' eine zweiwertige Gruppe ist,
- oder eine Gruppe R₈-O-CO-(CH₂)ₚ, wobei R₈ eine C₁₋₂₀-Kohlenwasserstoffgruppe ist und p eine ganze Zahl im Bereich von 1 bis 12 bedeutet;
- R₂ Wasserstoff, eine Gruppe vom Saccharidtyp, insbesondere (Glycosyl)ₙ, (Galactosyl)ₘ oder Sulfogalactosyl, einen Sulfatrest, einen Phosphatrest, eine Phosphorylethylamingruppe oder eine Phosphorylethylammoniumgruppe, wobei n eine ganze Zahl im Bereich von 1 bis 4 und m eine ganze Zahl im Bereich von 1 bis 8 ist;
- R₃ Wasserstoff oder eine gesättigte oder ungesättigte, gegebenenfalls hydroxylierte C₁₋₃₃-Kohlenwasserstoffgruppe, wobei die Hydroxygruppe(n) mit einer anorganischen Säure oder einer Säure R₇COOH, wobei R₇ die oben angegebenen Bedeutungen aufweist, verestert oder mit einer (Glycosyl)ₙ-Gruppe, (Galactosyl)ₘ-Gruppe, Sulfogalactosylgruppe, Phosphorylethylamingruppe oder einer Phosphorylethylammoniumgruppe verethert sein kann (können), und wobei die Gruppe R₃ auch mit einer oder mehreren C₁₋₁₄-Alkylgruppen substituiert sein kann;
- R₄ Wasserstoff, Methyl, Ethyl, eine gesättigte oder ungesättigte, geradkettige oder verzweigte, gegebenenfalls hydroxylierte C₃₋₅₀-Kohlenwasserstoffgruppe, eine Gruppe -CH₂-CHOH-CH₂-O-R₆, wobei R₆ eine C₁₀₋₂₆-Kohlenwasserstoffgruppe bedeutet, oder eine Gruppe R₈-O-CO-(CH₂)ₚ, wobei R₈ eine C₁₋₂₀-Kohlenwasserstoffgruppe bedeutet und p eine ganze Zahl im Bereich von 1 bis 12 ist;
- R₅ Wasserstoff oder eine gesättigte oder ungesättigte, geradkettige oder verzweigte, gegebenenfalls ein- oder mehrfach hydroxylierte C₁₋₃₀-Kohlenwasserstoffgruppe, wobei die Hydroxygruppe(n) mit einer (Glycosyl)ₙ-Gruppe, (Galactosyl)ₘ-Gruppe, Sulfogalactosylgruppe, Phosphorylethylamingruppe und einer Phosphorylethylammoniumgruppe verethert sein kann (können);
mit der Maßgabe, daß R₄ nicht Wasserstoff, Methyl oder Ethyl bedeutet, wenn R₃ und R₅ Wasserstoff bedeuten oder wenn R₃ Wasserstoff und R₅ Methyl bedeutet.

5. Verwendung nach Anspruch 4, **dadurch gekennzeichnet, daß** die Verbindung vom Ceramidtyp eine Verbindung der Formel (I) ist, worin bedeuten:
- R₁ entweder eine geradkettige oder verzweigte, gesättigte oder ungesättigte C₉₋₃₀-Kohlenwasserstoffgruppe, wobei die Gruppe mit einer oder mehreren Hydroxygruppen substituiert sein kann, die gegebenenfalls mit einer gesättigten oder ungesättigten C₁₆₋₃₀-Fettsäure verestert sind; oder eine Gruppe R"-(NR-CO)-R', wobei R Wasserstoff oder eine C₁₋₁₀-Kohlenwasserstoffgruppe mit einer oder mehreren Hydroxygruppen und vorzugsweise einer Hydroxygruppe bedeutet und R' und R" Kohlenwasserstoffgruppen sind, deren Summe an Kohlenstoffatomen im Bereich von 9 bis 30 liegt, wobei R' eine zweiwertige Gruppe ist;
- R₂ Wasserstoff, (Glycosyl)ₙ, (Galactosyl)ₘ oder Sulfogalactosyl, wobei n eine ganze Zahl im Bereich von 1 bis 4 und m eine ganze Zahl im Bereich von 1 bis 8 ist;
- R₃ Wasserstoff oder eine gesättigte oder ungesättigte C₁₆₋₂₇-Kohlenwasserstoffgruppe, wobei diese Gruppe mit einer oder mehreren C₁₋₁₄-Alkylgruppen substituiert sein kann; R₃ kann auch eine α-Hydroxyalkylgruppe mit 15 bis 26 Kohlenstoffatomen bedeuten, wobei die Hydroxygruppe gegebenenfalls mit einer α-Hydroxysäure mit 16 bis 30 Kohlenstoffatomen verestert sein kann;
- R₄ Wasserstoff, eine gesättigte oder ungesättigte C₁₆₋₂₇-Kohlenwasserstoffgruppe oder eine Gruppe -CH₂-CHOH-CH₂-O-R₆, wobei R₆ eine C₁₀₋₂₆-Kohlenwasserstoffgruppe bedeutet;
- R₅ Wasserstoff oder eine ein- oder mehrfach hydroxylierte C₁₋₄-Kohlenwasserstoffgruppe.

6. Verwendung nach Anspruch 4, **dadurch gekennzeichnet, daß** die Verbindung vom Ceramidtyp eine Verbindung der Formel (I) ist, worin R₁ eine gesättigte oder ungesättigte, von C₁₆₋₂₂-Fettsäuren abgeleitete, gegebenenfalls hydroxylierte Alkylgruppe, R₂ Wasserstoff und R₃ eine gesättigte, geradkettige, gegebenenfalls hydroxylierte C₁₅-Gruppe bedeutet.

7. Verwendung nach Anspruch 6, **dadurch gekennzeichnet, daß** die die Verbindung vom Ceramidtyp ausgewählt ist unter:
- N-Linoleoyldihydrosphingosin,
- N-Oleoyldihydrosphingosin,
- N-Palmitoyldihydrosphingosin,
- N-Stearoyldihydrosphingosin,
- N-Behenoyldihydrosphingosin,
- N-2-Hydroxypalmitoyldihydrosphingosin,
- N-Stearoylphytosphingosin, oder
den Gemischen dieser Verbindungen.

8. Verwendung nach Anspruch 7, **dadurch gekennzeichnet, daß** die Verbindung vom Ceramidtyp unter N-Oleoyldihydrosphingosin, N-2-Hydroxypalmitoyldihydrosphingosin und N-Stearoylphytosphingosin ausgewählt ist.

9. Verwendung nach einem der vorhergehenden Ansprüche, **dadurch gekennzeichnet, daß** die Zusammensetzung die Verbindung vom Ceramidtyp in einem Mengenanteil von 0,01 bis 10 Gew.-% enthält.

10. Verwendung nach einem der vorhergehenden Ansprüche, **dadurch gekennzeichnet, daß** die Zusammensetzung die Verbindung vom Ceramidtyp in einem Mengenanteil von 0,1 bis 1 Gew.-% enthält.

11. Verwendung nach einem der vorhergehenden Ansprüche, **dadurch gekennzeichnet, daß** die Zusammensetzung einen kosmetisch akzeptablen Träger enthält, der unter den organischen Lösungsmitteln, Wasser und/oder den Ölen ausgewählt ist.

12. Verwendung nach einem der vorhergehenden Ansprüche, **dadurch gekennzeichnet, daß** die Zusammensetzung ein filmbildendes Polymer enthält, das unter Nitrocellulose, Celluloseacetobutyrat, Polyvinylbutyralen, Alkydharzen, Polyestern, Acrylverbindungen und Polyurethanen ausgewählt ist.

13. Verwendung nach Anspruch 12, **dadurch gekennzeichnet, daß** die Zusammensetzung einen Weichmacher enthält.

14. Verwendung nach einem der vorhergehenden Ansprüche, **dadurch gekennzeichnet, daß** die Zusammensetzung in Form einer Zusammensetzung vorliegt, die auf die Nägel aufgetragen werden soll, beispielsweise als Nagellack oder eine Zusammensetzung zur Nagelpflege.

## Claims

1. Use, in a cosmetic composition, of at least one compound of ceramide type in order to make the outer surface of the nails uniform.

2. Use according to Claim 1, in order to improve the smooth nature of the outer surface of the nails.

3. Use according to Claim 1 or 2, in order to improve the shiny nature of the outer surface of the nails.

4. Use according to one of Claims 1 to 3, **characterized in that** the compound of ceramide type is a compound of formula (I) below: 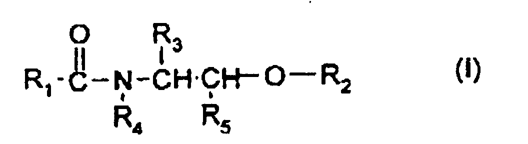 in which:
- R₁ denotes:
- either a saturated or unsaturated, linear or branched C₁-C₅₀, preferably C₅-C₅₀, hydrocarbon radical, it being possible for this radical to be substituted with one or more hydroxyl groups which are optionally esterified with an acid R₇COOH, R₇ being an optionally mono- or polyhydroxylated linear or branched, saturated or unsaturated C₁-C₃₅ hydrocarbon radical, it being possible for the hydroxyl or hydroxyls of the radical R₇ to be esterified with an optionally mono- or polyhydroxylated, linear or branched, saturated or unsaturated C₁-C₃₅ fatty acid,
- or a radical R"-(NR-CO)-R', R denotes a hydrogen atom or a mono- or polyhydroxylated, preferably monohydroxylated, C₁-C₂₀ hydrocarbon radical, R' and R" are hydrocarbon radicals, the sum of whose carbon atoms is between 9 and 30, R' being a divalent radical,
- or a radical R₈-O-CO-(CH₂)ₚ, R₈ denoting a C₁-C₂₀ hydrocarbon radical, p being an integer ranging from 1 to 12;
- R₂ is chosen from a hydrogen atom, a radical of saccharide type, in particular a (glycosyl)ₙ, (galactosyl)ₘ or sulphogalactosyl radical, a sulphate or phosphate residue, a phosphorylethylamine radical and a phosphorylethylammonium radical, in which n is an integer ranging from 1 to 4 and m is an integer ranging from 1 to 8;
- R₃ denotes a hydrogen atom or a C₁-C₃₃, saturated or unsaturated, hydroxylated or non-hydroxylated hydrocarbon radical, it being possible for the hydroxyl or hydroxyls to be esterified with an inorganic acid or an acid R₇COOH, R₇ having the same meanings as above, it being possible for the hydroxyl or hydroxyls to be etherified with a (glycosyl)ₙ, (galactosyl)ₘ, sulphogalactosyl, phosphorylethylamine or phosphorylethylammonium radical, it also being possible for R₃ to be substituted with one or more C₁-C₁₄ alkyl radicals;
- R₄ denotes a hydrogen atom, a methyl or ethyl radical, an optionally hydroxylated linear or branched, saturated or unsaturated C₃-C₅₀ hydrocarbon radical or a radical -CH₂-CHOH-CH₂-O-R₆ in which R₆ denotes a C₁₀-C₂₆ hydrocarbon radical or a radical Rₐ-O-CO- (CH₂)ₚ, R₈ denotes a C₁-C₂₀ hydrocarbon radical, p being an integer ranging from 1 to 12,
- R₅ denotes a hydrogen atom or an optionally mono- or polyhydroxylated linear or branched, saturated or unsaturated C₁-C₃₀ hydrocarbon radical, it being possible for the hydroxyl or hydroxyls to be etherified with a (glycosyl)ₙ, (galactosyl)ₘ, sulphogalactosyl, phosphorylethylamine or phosphorylethylammonium radical,
with the proviso that when R₃ and R₅ denote hydrogen or when R₃ denotes hydrogen and R₅ denotes methyl, then R₄ does not denote a hydrogen atom or a methyl or ethyl radical.

5. Use according to Claim 4, **characterized in that** the compound of ceramide type is a compound of formula (I) in which
- R₁ denotes either a saturated or unsaturated, linear or branched C₉-C₃₀ hydrocarbon radical, it being possible for this radical to be substituted with one or more hydroxyl groups, these hydroxyl groups optionally being esterified with a saturated or unsaturated C₁₆-C₃₀ fatty acid; or a radical R"-(NR-CO)-R', where R denotes a hydrogen atom or a mono- or polyhydroxylated, preferably monohydroxylated, C₁-C₁₀ hydrocarbon radical, R' and R" are hydrocarbon radicals, the sum of whose carbon atoms is between 9 and 30, R' being a divalent radical;
- R₂ denotes a hydrogen atom or a (glycosyl)ₙ, (galactosyl)ₘ or sulphogalactosyl radical, in which n is an integer ranging from 1 to 4 inclusive and m is an integer ranging from 1 to 8 inclusive;
- R₃ denotes a hydrogen atom or a saturated or unsaturated C₁₆-C₂₇ hydrocarbon radical, it being possible for this radical to be substituted with one or more C₁-C₁₄ alkyl radicals; R₃ may also denote a C₁₅-C₂₆ α-hydroxyalkyl radical in which the hydroxyl group may optionally be esterified with a C₁₆-C₃₀ α-hydroxy acid;
- R₄ denotes a hydrogen atom, a saturated or unsaturated C₁₆-C₂₇ hydrocarbon radical or a radical -CH₂-CHOH-CH₂-O-R₆ in which R₆ denotes a C₁₀-C₂₆ hydrocarbon radical;
- R₅ denotes a hydrogen atom or a mono- or polyhydroxylated C₁-C₄ hydrocarbon radical.

6. Use according to Claim 4, **characterized in that** the compound of ceramide type is a compound of formula (I) in which R₁ denotes an optionally hydroxylated saturated or unsaturated alkyl radical derived from C₁₆-C₂₂ fatty acids; R₂ denotes a hydrogen atom; and R₃ denotes an optionally hydroxylated, saturated, linear C₁₅ radical.

7. Use according to Claim 6, **characterized in that** the compound of ceramide type is chosen from:
- N-linoleoyldihydrosphingosine,
- N-oleoyldihydrosphingosine,
- N-palmitoyldihydrosphingosine,
- N-stearoyldihydrosphingosine,
- N-behenoyldihydrosphingosine,
- N-2-hydroxypalmitoyldihydrosphingosine,
- N-stearoylphytosphingosine, or mixtures of these compounds.

8. Use according to Claim 7, **characterized in that** the compound of ceramide type is chosen from N-oleoyldihydrosphingosine, N-2-hydroxypalmitoyldihydrosphingosine and N-stearoylphytosphingosine.

9. Use according to any one of the preceding claims, **characterized in that** the composition comprises a content of ceramide-type compound of from 0.01% to 10% by weight.

10. Use according to any one of the preceding claims, **characterized in that** the composition comprises a content of ceramide-type compound of from 0.1% to 1% by weight.

11. use according to any one of the preceding claims, **characterized in that** the composition comprises a cosmetically acceptable support chosen from organic solvents, water and/or oils.

12. Use according to any one of the preceding claims, **characterized in that** the composition comprises a film-forming polymer chosen from nitrocellulose, cellulose acetobutyrate, polyvinyl butyrals, alkyd resins, polyesters, acrylics and polyurethanes.

13. Use according to Claim 12, **characterized in that** the composition comprises a plasticizer.

14. Use according to any one of the preceding claims, **characterized in that** the composition is in the form of a composition to be applied to the nail, such as a nail varnish or a nail care composition.
